Europäisches Patentamt

(19) European Patent Office

Office européen des brevets

(11) Numéro de publication : **0 337 990 B1**

(12) **FASCICULE DE BREVET EUROPEEN**

(45) Date de publication du fascicule du brevet :
25.09.91 Bulletin 91/39

(51) Int. Cl.⁵ : **A63B 23/00**

(21) Numéro de dépôt : **87906656.1**

(22) Date de dépôt : **22.10.87**

(86) Numéro de dépôt international :
**PCT/CH87/00146**

(87) Numéro de publication internationale :
**WO 89/03707 05.05.89 Gazette 89/10**

⑤④ **APPAREIL DE RESISTANCE A L'EXPIRATION DESTINE A AMELIORER LA VENTILATION PULMONAIRE.**

(43) Date de publication de la demande :
25.10.89 Bulletin 89/43

(45) Mention de la délivrance du brevet :
25.09.91 Bulletin 91/39

(84) Etats contractants désignés :
**AT BE CH DE FR GB IT LI LU NL SE**

(56) Documents cités :
**DE-U- 8 625 936**
**FR-A- 2 236 527**
**US-A- 3 908 987**

(73) Titulaire : **VARIORAW PERCUTIVE S.A.**
**Rue de Trévelin 30**
**CH-1170 Aubonne (CH)**

(72) Inventeur : **LIARDET, Claude**
**30, rue Trévelin**
**CH-1170 Aubonne (CH)**

(74) Mandataire : **Ganguillet, Cyril et al**
**ABREMA Agence Brevets & Marques**
**Ganguillet & Humphrey Rue Centrale 5 C.P.**
**2065**
**CH-1002 Lausanne (CH)**

EP 0 337 990 B1

## Description

La présente invention a pour objet un appareil de résistance à l'expiration destiné à améliorer la ventilation pulmonaire.

On connaît divers appareils destinés à une thérapie spécifique du domaine respiratoire en améliorant la ventilation périphérique chez des malades ayant une respiration perturbée en raison de causes diverses, comme par exemple, bronchite chronique, asthme, problème de compliance, rapport de pression pleurale par rapport à la pression alvéolaire inadéquat, état de surinfection, hypersécrétion et bouchons muqueux, etc. Ces appareils sont utilisés avec succès, toutefois, ils sont tous d'une conception complexe, en général fort encombrants et fort coûteux.

On a déjà réalisé des appareils de petites dimensions comportant un passage d'air horizontal relié à un passage d'air vertical comportant un orifice d'échappement, un élément mobile étant disposé de façon à obstruer l'orifice d'échappement avant expiration et à opposer une résistance à l'expiration sous l'action d'un organe élastique ou par l'effet de son poids propre. On a notamment réalisé un appareil dans lequel une bille pesante est disposée dans un canal d'échappement en forme d'entonnoir vertical, la bille libre de se déplacer verticalement se soulevant de son support lors de l'expiration et opposant une résistance à l'expiration par l'effet de son poids propre.

Un appareil de ce type est décrit dans le brevet américain US-A-3,908,987. Cet appareil comprend une première partie de forme tubulaire comportant un orifice d'entrée d'air dans lequel le patient peut expirer, et une seconde partie perpendiculaire à la première, comportant un canal d'échappement de forme conique circulaire à axe vertical. Une bille sphérique de diamètre supérieur au plus petit diamètre du canal d'échappement est disposée dans le canal d'échappement, de façon à obstruer ledit canal avant expiration. La bille peut se déplacer librement dans ce canal et oppose une résistance à l'expiration de l'air expiré par le patient par l'effet de son poids propre. L'appareil comporte un couvercle muni d'un trou pour permettre à l'air expiré de s'échapper, les dimensions du trou étant inférieures à celles de la bille.

Les résultats obtenus avec ce type d'appareil ne sont toutefois pas aussi bons que ceux obtenus avec les appareils plus complexes mentionnés plus haut. Des tests cliniques ont montré que l'effet de percussion obtenu avec ce type d'appareil reste faible et difficilement contrôlable.

Un autre type d'appareil de petites dimensions est décrit dans la demande de brevet française FR-A-2,236,527. Cet appareil comprend un canal en forme de gouttière, d'inclinaison réglable et comportant à l'une de ses extrémités un ajutage dont l'axe est sensiblement parallèle à l'axe du canal. L'ajutage est connecté à un tuyau souple dans lequel le patient doit expirer. La conception de cet appareil est très rudimentaire et ne lui permet pas d'obtenir des résultats comparables à ceux des appareils plus complexes. De plus, cet appareil nécessite d'être disposé sur un support horizontal tel qu'une table et ne peut pas être tenu dans la main.

Le but de la présente invention est de proposer un appareil qui permette d'obtenir des résultats équivalents à ceux obtenus avec les appareils de conception plus complexe, qui soit de petites dimensions, simple et peu coûteux, et qui puisse facilement être transporté, par exemple dans une poche.

A cet effet, l'appareil de résistance à l'expiration selon l'invention présente les caractéristiques spécifiées dans la revendication 1. Des caractéristiques correspondant à des formes d'exécution particulières de l'appareil de résistance sont spécifiées dans les revendications subordonnées à la revendication 1.

Les études et les essais cliniques effectués ont permis de mettre en évidence l'importance de l'inclinaison de l'axe du canal conique de façon à constituer d'une part un lit pour le déplacement de la bille et d'autre part une butée à ce déplacement de façon à obstruer le canal, ce qui permet à la bille de garder un contact permanent avec son support et d'obtenir un effet de percussion notablement augmenté par rapport à l'effet obtenu avec les appareils décrits plus haut, tout en recherchant une basse fréquence.

D'autres avantages et caractéristiques favorables de l'appareil de résistance à l'expiration selon l'invention ressortiront plus clairement de la description donnée ci-après, à titre d'exemple, en se référant au dessin annexé dans lequel :

la fig. 1 est une coupe verticale d'un premier exemple d'appareil de résistance à l'expiration,
la fig. 2 est une coupe verticale d'un deuxième exemple d'appareil de résistance à l'expiration, et
la fig. 3 représente un troisième exemple d'appareil de résistance, vu de l'avant sur la figure 3a et vu de côté sur la figure 3b.

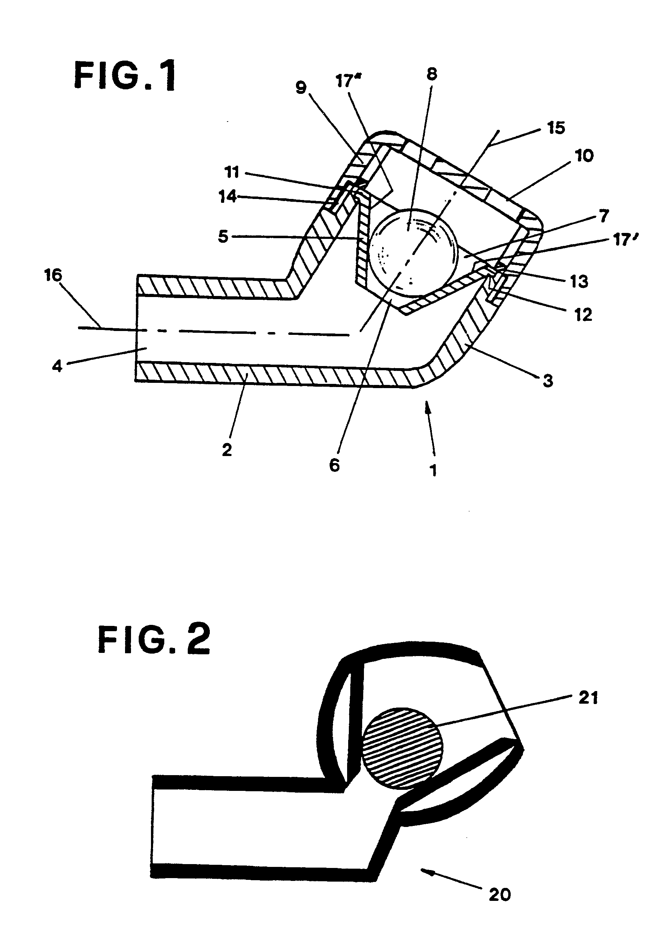

Comme représenté sur la figure 1, l'appareil se compose d'un premier élément en forme de tube cylindrique coudé 1, ouvert à ses deux extrémités, comportant une première partie tubulaire rectiligne 2 destinée à être tenue en position horizontale et une seconde partie tubulaire 3 inclinée vers le haut par rapport à la première partie. La première partie tubulaire 2 comporte un orifice d'entrée d'air 4 dans lequel le patient peut expirer.

2

Un élément creux 5 dont les parois ont la forme d'un cône droit de révolution tronqué est disposé à l'intérieur de la seconde partie tubulaire 3, de façon que son extrémité de plus grand diamètre 7 soit située au niveau de l'extrémité de la seconde partie tubulaire 3, l'extrémité de plus petit diamètre 6 de l'élément en forme de cône étant disposée à l'intérieur de l'élément tubulaire. Une bille 8 dont le diamètre est supérieur au plus petit diamètre 6 de l'élément conique est simplement posée contre la paroi interne de l'élément conique et obstrue totalement le canal constitué par l'élément conique, avant que le patient n'expire. L'appareil comporte en outre un couvercle 9 disposé à l'extrémité de la seconde partie tubulaire 3 et destiné à empêcher la bille 8 de s'échapper. Afin de permettre à l'air expiré de s'échapper de l'appareil, le couvercle comporte une ou plusieurs ouvertures 10 conformées de façon à empêcher le passage de la bille. La fixation du couvercle 9 sur l'élément tubulaire est réalisée par un dispositif à baïonnette 14. Bien entendu ce dispositif de fixation peut être remplacé par un dispositif de clipsage ou être conçu de façon que le couvercle soit vissé sur l'élément tubulaire.

L'élément conique 5 est fixé de façon amovible dans l'appareil. A cet effet, il comporte un rebord extérieur 11 situé sur le pourtour de son extrémité de plus grand diamètre, ledit rebord étant destiné à être logé dans une encoche correspondante 12 réalisée sur le pourtour de la paroi intérieure de l'extrémité de l'élément tubulaire. Le blocage en position de l'élément conique est réalisé lors de la mise en place du couvercle grâce à l'action d'un organe 13 en forme de couronne qui fait saillie à l'intérieur du couvercle sur tout son pourtour et qui s'appuie contre l'extrémité de grand diamètre de l'élément conique. La conception structurelle démontable de l'appareil qui peut être facilement séparé en quatre parties, à savoir l'élément tubulaire coudé, l'élément conique, le couvercle et la bille, permet un nettoyage efficace et son utilisation dans des conditions d'hygiène optimales.

Afin d'obtenir l'effet de percussion souhaité lors de l'expiration du patient, il est nécessaire d'éviter que la bille ne se soulève et flotte au dessus de son support, mais qu'elle reste en contact avec celui-ci. Cette caractéristique est obtenue par l'inclinaison vers le haut d'un angle inférieur à 90° de l'axe 15 de l'élément conique par rapport à l'axe 16 à la première partie 2 de l'élément tubulaire coudé. En outre, l'angle d'ouverture de l'élément conique doit être choisi de façon que l'angle formé par une génératrice 17' ou 17" de la paroi du canal et l'axe 15 dudit canal soit inférieur à l'angle d'inclinaison de l'axe du canal par rapport à l'axe 16 de la première partie tubulaire 2. De cette façon, la bille va se déplacer en roulant sur la paroi intérieure de l'élément conique, la partie la plus basse de ladite paroi 17' constituant un lit de roulement pour la bille, la partie la plus haute de ladite paroi 17" constituant une butée au mouvement de la bille. Lorsque la première partie 2 de l'élément tubulaire est maintenue en position horizontale par le patient, avant expiration la bille obstrue le canal conique par l'effet de gravité dû à son poids. Lors de l'expiration, la position instantanée de la bille résulte d'un état d'équilibre entre la pression de l'air expiré par le patient et la force de gravité due au poids de la bille. La caractéristique d'amortissement de la bille étant très faible, il s'ensuit un mouvement oscillatoire de cette dernière engendrant une pression variable qui s'oppose à l'expiration, en constituant une résistance oscillante positive à l'expiration.

L'expérience a montré que cet appareil permet une ventilation en percussion d'une grande efficacité, des sécrétions étant mobilisées en quelques minutes dans l'arbre bronchique du patient, permettant leur expectoration aisée. Les meilleurs résultats sont obtenus avec une expiration lente et aussi complète que possible. Des mesures par ballonnets oesophagiens ont montré que le phénomène de percussion atteint le niveau périphérique du poumon. Outre son coût très modeste, cet appareil présente le grand avantage de pouvoir être emporté facilement dans la poche du patient lors de tous ses déplacements. Son utilisation à titre de thérapie par entraînement constitue une très bonne gymnastique respiratoire.

Les meilleurs résultats sont obtenus avec un appareil dans lequel l'inclinaison de l'axe 15 du canal conique par rapport à l'axe 16 de la première partie 2 de l'élément tubulaire est comprise entre 50° et 70°, et en utilisant un élément de cône tronqué dont la pente de la paroi 17' (ou 17") du cône forme un angle compris entre 20° et 30° par rapport à l'axe 15 dudit cône. En particulier, d'excellents résultats sont obtenus avec un appareil dont l'axe 15 de l'élément de cône est incliné de 60° vers le haut par rapport à l'axe 16 de la première partie 2 de l'élément tubulaire, l'angle formé par la direction de la paroi 17' (ou 17") dudit canal conique avec son axe 15 étant de 30°. Dans une forme d'exécution préférentielle de l'appareil, l'élément tubulaire 1, l'élément de cône 5 et le couvercle 9 sont réalisés en matière plastique par moulage. On utilisera de préférence des billes en acier inox ayant un diamètre compris entre 16 et 25 millimètres. Dans ce cas, la longueur du canal conique devrait être d'au moins 1 centimètre et le diamètre de sa plus petite extrémité d'au moins 1 centimètre. Afin de permettre un bon fonctionnement de l'appareil, la section du ou des orifices 10 de sortie d'air situées dans le couvercle devrait être égale ou supérieure à la section d'entrée 6 de l'élément de cône.

Bien entendu l'appareil fonctionne également pour d'autres dimensions et en utilisant d'autres matières pour ses éléments constitutifs, pour autant que le poids de la bille soit suffisant pour que l'on obtienne l'effet de percussion souhaité.

Les études cliniques entreprises avec cet appareil de résistance ont permis de mettre en évidence les phénomènes suivants :

3

— une résistance positive linéaire à l'expiration adaptée aux valeurs thérapeutiques connues,

— une résistance percutante (différence de pression$\Delta$P de l'ordre de 5 à 10 centimètres d'eau) et de basse fréquence (environ 10 à 20 $\Delta$P par seconde) adaptée aux valeurs thérapeutiques connues, c'est-à-dire correspondant aux valeurs intrathoraciques d'un "clapping" moyen.

Ces études ont également montré que cet appareil permet une régulation automatique à la fois de la pression linéaire positive, de la percussion ($\Delta$P) et de la fréquence de cette percussion, cette régulation étant inhérente au système et non dépendante du débit d'air.

On a représenté à la figure 2 un deuxième exemple d'exécution de l'appareil de résistance. Cet appareil 20 est également réalisé en matière plastique et comporte une partie amovible non représentée permettant l'introduction de la bille 21.

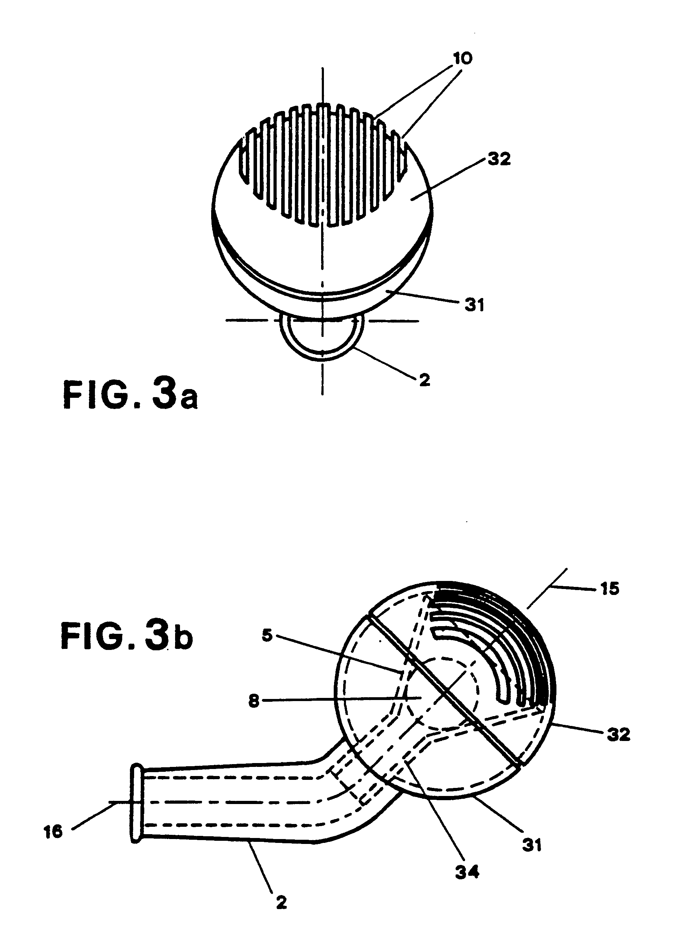

Un troisième exemple d'exécution de l'appareil de résistance est représenté à la figure 3. La première partie tubulaire 2 de cet appareil est coudée à son extrémité opposée à l'orifice d'entrée et est surmontée d'une partie 31 en forme de demi-sphère comportant une ouverture circulaire à sa base permettant de communiquer avec le canal de la première partie tubulaire 2. L'élément du canal conique 5 est prolongé à sa base par une partie tubulaire cylindrique 34 qui vient se ficher dans le canal de la première partie tubulaire 2. Un couvercle 32 en forme de demi-sphère comportant des ouvertures 10 permet de fermer l'appareil et d'empêcher l'échappement de la bille 8.

Bien entendu, de nombreuses variantes d'exécution peuvent être envisagées, notamment quant à l'aspect esthétique de l'appareil. Il est possible de donner à cet appareil une forme extérieure présentant l'empreinte d'un ou plusieurs doigts destinée à une bonne tenue en main dudit appareil.

## Revendications

1. Appareil de résistance à l'expiration destiné à améliorer la ventilation pulmonaire, comprenant une première partie de forme tubulaire (2) comportant un orifice d'entrée d'air (4) dans lequel le patient peut expirer, et une seconde partie (3) comportant un canal d'échappement (5) de forme conique circulaire, une bille sphérique (8) de diamètre supérieur au plus petit diamètre du canal d'échappement étant disposée dans ledit canal de façon à obstruer ledit canal avant expiration, la bille pouvant se déplacer librement dans ledit canal et opposant une résistance à l'expiration de l'air expiré par le patient par l'effet de son poids propre, l'appareil étant conformé de façon à permettre à l'air expiré de s'échapper par au moins un trou situé dans une zone opposée à l'orifice d'entrée dudit canal, les dimensions du trou étant choisies de façon à empêcher l'échappement de la bille, caractérisé en ce que l'axe (15) du canal est incliné vers le haut par rapport à l'axe (16) de la partie de forme tubulaire d'un angle d'inclinaison compris entre 30° et 80°, l'angle formé par une génératrice (17', 17") de la paroi du canal et l'axe (15) du canal étant inférieur à l'angle d'inclinaison de l'axe du canal.

2. Appareil selon la revendication 1, caractérisé en ce que l'angle formé par une génératrice de la paroi du canal conique et l'axe du canal est compris entre 20° et 35°.

3. Appareil selon la revendication 1, caractérisé en ce que l'inclinaison de l'axe du canal conique par rapport à l'axe de la première partie de forme tubulaire est de 60°, l'angle formé par une génératrice de la paroi du canal et l'axe du canal étant de 30°.

4. Appareil selon l'une des revendications précédentes, caractérisé en ce qu'il comprend un élément de forme tubulaire coudé (1) ouvert à ses deux extrémités, la première extrémité (4) constituant l'orifice d'entrée d'air dans lequel le patient peut expirer, un élément de couvercle (9) comportant au moins une ouverture (10) étant fixé de façon amovible à la seconde extrémité de l'élément coudé, le canal conique étant constitué par un élément (5) en forme de cône tronqué, également fixé de façon amovible à la seconde extrémité de l'élément coudé, l'extrémité (6) de plus petit diamètre de l'élément de cône étant située à l'intérieur de l'élément coudé.

5. Appareil selon l'une des revendications précédentes, caractérisé en ce que la bille est en acier, son diamètre étant compris entre 16 et 25 millimètres.

6. Appareil selon l'une des revendications précédentes, caractérisé en ce que la longueur du canal conique est d'au moins 1 centimètre, le diamètre de sa plus petite extrémité étant d'au moins 1 centimètre.

## Patentansprüche

1. Widerstandsgerät gegen das Ausatmen zur Verbesserung der Lungenventilation, mit einem ersten rohrförmigen Abschnitt (2), der eine Lufteinlaßöffnung (4) aufweist, in die der Patient ausatmen kann, und mit einem zweiten Abschnitt (3), der einen kreisförmigen konischen Auslaßkanal (5) aufweist, in dem eine Kugel (8), deren Durchmesser größer ist als der kleinste Durchmesser des Kanals, derart angeordnet ist, daß sie den Kanal vor dem Ausatmen verschließt, wobei sich die Kugel frei im Kanal bewegen kann und dem Ausatmen der vom Patienten ausgeatmeten Luft aufgrund ihres Eigengewichts einen Widerstand entgegensetzt, wobei das Gerät derart ausgestaltet ist, daß es eine Entweichen der ausgeatmeten Luft durch wenigstens eine Öffnung ermöglicht, die in einem Bereich gegenüber der Einlaßöffnung des Kanals vorgesehen ist und wobei die Abmessungen der Öffnung derart gewählt sind, daß ein Austreten der Kugel verhindert wird, dadurch gekennzeichnet, daß die Achse (15) des Kanals nach oben bezüglich der Achse (16) des rohrförmigen Abschnitts um einen Winkel geneigt ist, der zwischen 30° und 80° liegt und daß der Winkel, der durch eine Erzeugende (17', 17") der Wand des Kanals und der Achse (15) des Kanals gebildet wird, kleiner ist als der Neigungswinkel der Achse des Kanals.

2. Gerät nach Anspruch 1, dadurch gekennzeichnet, daß der durch eine Erzeugende der Wand des konischen Kanals und der Achse des Kanals gebildete Winkel zwischen 20° und 35° liegt.

3. Gerät nach Anspruch 1, dadurch gekennzeichnet, daß die Neigung der Achse des konischen Kanals bezüglich der Achse des ersten rohrförmigen Abschnitts 60° beträgt und daß der Winkel, der durch die Erzeugende der Wand des Kanals und der Achse des Kanals gebildet wird, 30° beträgt.

4. Gerät nach einem der vorhergehenden Ansprüche, dadurch gekennzeichnet, daß es ein gebogenes rohrförmiges Teil (1) aufweist, das an seinen beiden Enden offen ist, wobei das erste Ende (4) die Lufteinlaßöffnung bildet, in welche der Patient ausatmet, daß ein Deckelteil 9 wenigstens eine Öffnung (10) aufweist und lösbar mit dem zweiten Ende des gebogenen Teils verbunden ist, daß der konische Kanal aus einem kegelstumpfförmigen Teil (5) besteht, das ebenfalls lösbar mit dem zweiten Ende des gebogenen Teils verbunden ist und daß das Ende (6) mit dem kleineren Durchmesser des kegelstumpfförmigen Teils im Inneren des gebogenen Teils angeordnet ist.

5. Gerät nach einem der vorhergehenden Ansprüche, dadurch gekennzeichnet, daß die Kugel aus Stahl besteht und daß ihr Durchmesser zwischen 16 und 25 Millimetern beträgt.

6. Gerät nach einem der vorhergehenden Ansprüche, dadurch gekennzeichnet, daß die Länge des konischen Kanals wenigstens ein Zentimeter beträgt und daß der Durchmesser an seinem kleineren Ende wenigstens ein Zentimeter beträgt.

## Claims

1. Expiration-resisting apparatus designed for improving pulmonary ventilation, comprising a first part having a tubular shape (2) comprising an air inlet aperture (4) into which the patient may expire, and a second part (3) comprising an exhaust channel (5) having a circular cone shape, a spherical ball (8) having a diameter greater than the smallest diameter of the exhaust channel being disposed in said channel so as to obstruct said channel before expiration, with the ball being able to move freely in said channel and opposing a resistance to the expiration of air expired by the patient by the effect of its own weight, the apparatus being designed so as to allow the expired air to escape through at least one hole located in a zone opposite to the inlet aperture of said channel, with the dimensions of the hole being chosen so as to prevent the escape of the ball, characterised in that the axis (15) of the channel is inclined upwards with respect to the axis (16) of the tubular-shaped part by an angle of inclination of between 30° and 80°, with the angle formed by a generator (17', 17") of the wall of the channel and the axis (15) of the channel being less than the angle of inclination of the axis of the channel.

2. Apparatus according to claim 1, characterised in that the angle formed by a generatrix of the wall of the conical channel and the axis of the channel lies between 20° and 35°.

3. Apparatus according to claim 1, characterised in that the inclination of the axis of the conical channel with respect to the axis of the first tubular-shaped part is 60°, with the angle formed by a generatrix of the wall of the channel and the axis of the channel being 30°.

4. Apparatus according to one of the preceding claims, characterised in that it comprises an element having a bent tubular shape (1) open at its two ends, with the first end (4) forming the air inlet orifice into which the patient may expire, a cover element (9) comprising at least one aperture (10) being fixed in a detachable manner to the second end of the bent element, with the conical channel being formed by an element (5) in the form of a truncated cone, also detachably fixed to the second end of the bent element, with the end (6) of the conical element having the smaller diameter being located inside the bent element.

5. Apparatus according to one of the preceding claims, characterised in that the ball is made of steel, with its diameter being between 16 and 25 millimetres.

6. Apparatus according to one of the preceding claims, characterised in that the length of the conical channel is at least 1 centimetre, with the diameter of its smallest end being at least 1 centimetre.

# FIG.1

# FIG.2

FIG. 3a

FIG. 3b